# EUROPÄISCHE PATENTSCHRIFT

(11) **EP 4 568 604 B1**
(45) Veröffentlichungstag und Bekanntmachung des Hinweises auf die Patenterteilung: **08.10.2025**
(21) Anmeldenummer: 24794749.2
(22) Anmeldetag: 22.10.2024
(51) Int. Cl.: A61B 18/14

(54) **CHIRURGISCHES INSTRUMENT**
SURGICAL INSTRUMENT
INSTRUMENT CHIRURGICAL

(30) Priorität: 24.10.2023 DE 102023129225
(43) Veröffentlichungstag der Anmeldung: 18.06.2025
(73) Patentinhaber: Aesculap AG, 78532 Tuttlingen (DE)
(72) Erfinder: CIK, Justin Danny, 78052 Villingen (DE); HAFNER, Nikolaus, 78532 Tuttlingen (DE); HEIZMANN, Patrick, 78183 Hüfingen (DE); MASER, Thomas, 78166 Donaueschingen (DE); TEODOROVIC, Boris, 78532 Tuttlingen (DE); SCHWEITZER, Tom, 78532 Tuttlingen (DE); MELCER, Marco, 72469 Meßstetten (DE)
(74) Vertreter: Winter, Brandl - Partnerschaft mbB
(86) Internationale Anmeldenummer: PCT/EP2024/079781
(87) Internationale Veröffentlichungsnummer: WO 2025/087883

(56) Entgegenhaltungen:
- EP-A1- 1 747 761
- DE-A1- 102023 132 370
- US-A1- 2015 196 352
- US-A1- 2020 100 833
- US-A1- 2021 196 336
- US-A1- 2021 369 333

## Beschreibung

### Technisches Gebiet

Die vorliegende Offenbarung betrifft ein medizinisches Hochfrequenz-Chirurgie Instrument (HF-Instrument), insbesondere ein bipolares Gefäßversiegelungsinstrument, mit einem Maulteil und zumindest einer Metallelektrode zum Versiegeln von Gewebe.

### Hintergrund der Offenbarung

Bei der Hochfrequenz-Chirurgie (auch als HF-Chirurgie bezeichnet) wird ein hochfrequenter Wechselstrom durch den menschlichen Körper oder ein Körperteil geleitet, um Gewebe durch die damit verursachte Erwärmung gezielt zu veröden (Koagulation) bzw. zu schneiden (Elektrotomie). Das so geschädigte Gewebe wird später vom umgebenden gesunden Gewebe resorbiert. Ein wesentlicher Vorteil gegenüber herkömmlicher Schneidetechnik mit dem Skalpell ist, dass gleichzeitig mit dem Schnitt eine Blutungsstillung durch Verschluss der betroffenen Gefäße erfolgen kann, im Sinne einer Koagulation.

Derzeit wird am häufigsten die monopolare HF-Technik in der HF-Chirurgie angewendet. Dabei wird ein Pol der HF-Spannungsquelle über eine möglichst großflächige Gegenelektrode mit dem Patienten verbunden, beispielsweise durch Kontakte auf dem Operationstisch, auf dem der Patient liegt, durch Kontaktarmbänder bzw. Kontaktfußbänder oder durch Klebeelektroden. Diese Gegenelektrode nennt man oft neutrale Elektrode bzw. Neutralelektrode. Der andere Pol wird an das chirurgische Instrument angeschlossen und dieses bildet die sogenannte aktive Elektrode bzw. Aktivelektrode. Der Strom fließt über den Weg des geringsten Widerstandes von der Aktivelektrode zur Neutralelektrode. In unmittelbarer Nähe der Aktivelektrode ist die Stromdichte am höchsten, hier findet der thermische Effekt am stärksten statt. Die Stromdichte nimmt mit dem Quadrat des Abstands ab. Die neutrale Elektrode sollte möglichst großflächig und mit dem Körper gut verbunden sein, sodass die Stromdichte im Körper geringgehalten wird und keine Verbrennungen stattfinden. Die Haut an der Neutralelektrode wird durch die große Fläche nicht spürbar erwärmt. Bei der Anbringung der Neutralelektrode gelten strenge Sicherheitsmaßnahmen. Um keine Verbrennungen zu verursachen, sind richtige Position und guter Kontakt der neutralen Elektrode (abhängig vom Operationsgebiet) ausschlaggebend.

Bei der bipolaren HF-Technik fließt der Strom im Gegensatz zur monopolaren Technik durch einen kleinen Teil des Körpers - denjenigen, in dem die chirurgische Wirkung (Schnitt oder Koagulation) gewünscht ist. Zwei gegeneinander isolierte Metallelektroden, welche in Maulteilen des HF-Instruments aufgenommen sind und zwischen denen die HF-Spannung anliegt, werden direkt an die Operationsstelle geführt. Der Stromkreis wird über das dazwischenliegende Gewebe geschlossen. In dem Gewebe zwischen den Metallelektroden findet der thermische Effekt statt.

Bei derartigen HF-Instrumenten, insbesondere bei bipolaren Versiegelungsinstrumenten sind die Maulteile bevorzugt in einer Sandwichbauweise hergestellt/ ausgebildet. Die Maulteile bestehen aus der oder haben die dünne Metallelektrode, welche als Kontaktfläche zu dem Gewebe fungiert, einem Abstandshalter aus Kunststoff, der für die elektrische und thermische Isolation sorgt, und einem Trägerbauteil, welches vorgesehen und ausgebildet ist, eine Krafteinleitung zu gewährleisten und eine Schließmechanik beinhaltet. Das Trägerbauteil verleiht dem Maulteil die notwendige Stabilität und Steifigkeit.

Eine derartiges, in Sandwichbauweise ausgebildetes Maulteil ist jedoch in einem Herstellungsprozess aufwendig und kostenintensiv. Durch die verschiedenen Bauteile, welche miteinander verbunden werden müssen, kommt es zu einer Aufsummierung von Fertigungstoleranzen, was eine Passgenauigkeit und damit eine Qualität des Maulteils bzw. des HF-Instruments reduziert.

Ein Ansatz, um diese Nachteile zu beheben, ist, die Metallelektrode bzw. eine Branche des Maulteils als ein massives Bauteil auszulegen/ auszubilden.

Eine derartige massive Metallelektrode hat jedoch eine hohe thermische Masse, was insbesondere bei größeren Maulteilen dazu führt, dass ein Großteil einer der Metallelektrode durch einen HF-Generator zugeführten Energie nicht in die Gewebeversiegelung, sondern in die Erwärmung der massiven Metallelektrode fließt.

Eine zu große Kühlmasse an einer Rückseite der Elektroden, könnte also der Metallelektrode und auch dem Gewebe so viel Wärmeenergie entziehen, dass die Versiegelung/der Versiegelungsvorgang länger dauern würde, als unbedingt notwendig ist. Gleichzeitig, soll das Maulteil einen Kühlkörper aufweisen, der die Metallelektrode abkühlen kann, wenn diese nicht im Einsatz ist. So können ungewollte Schädigungen von umliegendem Gewebe minimiert werden, beispielsweise bei Berührungen während der Präparation von Gewebe oder bei Positionsänderungen des Maulteils.

Aus der US 2020 / 100 833 A1 ist ein HF-Instrument mit einem Maulteil mit zwei Schenkeln bekannt. Die zwei Schenkel sind relativ zueinander zwischen einem geöffneten Zustand und einem geschlossenen Zustand des Maulteils bewegbar. Das Maulteil weist zumindest eine Metallelektrode mit einer Kontaktfläche zum Kontaktieren von Gewebe auf. Das Instrument hat einen Kühlkörper. Die zumindest eine Metallelektrode und der Kühlkörper stehen in einer wärmeleitenden Verbindung zueinander.

Die US 2015 / 196 352 A1, die US 2021 / 369 333 A1, die US 2021 / 196 336 A1 und die EP 1 747 761 A1 offenbaren jeweils ein Maulteil mit einem Kühlkörper für eine Elektrode.

### Kurzbeschreibung der Offenbarung

Die Aufgabe der vorliegenden Offenbarung besteht also darin, die Nachteile des Stands der Technik zu überkommen oder zumindest zu mindern und insbesondere ein chirurgisches Instrument bereitzustellen, bei dem eine Elektrode sowohl effektiv erhitzt als auch gekühlt werden kann. Insbesondere sollten zu große Wärmeverluste durch die Kühlung vermieden werden.

Diese Aufgabe wird durch ein chirurgisches Instrument gemäß den Merkmalen des Anspruchs 1 gelöst.

Die vorliegende Offenbarung betrifft ein chirurgisches (Hochfrequenz-)Instrument mit einem Maulteil oder einem distalen Effektor mit zwei Schenkeln bzw. Armen, die relativ zueinander zwischen einem geöffneten Zustand / Freigabezustand und einem geschlossenen Zustand / Klemmzustand des Maulteils bewegbar sind. Das Maulteil weist zumindest eine (Metall-)Elektrode mit einer Kontaktfläche zum Kontaktieren von Gewebe und einen von der Kontaktfläche abgewandten Kühlkörper auf. In dem geschlossenen Zustand des Maulteils sind die zumindest eine Metallelektrode und der zumindest eine Kühlkörper voneinander beabstandet und thermisch getrennt und in dem offenen Zustand des Effektors stehen die zumindest eine Metallelektrode und der zumindest eine Kühlkörper in einer (wärmeleitenden / wärmeübertragenden / thermisch gekoppelten) Verbindung miteinander.

Das Maulteil ist wie eine Schere zwischen dem offenen Zustand und dem geschlossenen Zustand bewegbar. Das Maulteil hat dabei insbesondere die zwei relativ zueinander bewegbaren Arme, die auf und zugemacht werden können. In dem geschlossenen Zustand hat die Kontaktfläche Kontakt mit dem Gewebe und in dem offenen Zustand ist die Kontaktfläche von dem Gewebe beabstandet. Die zumindest eine Metallelektrode und der zumindest eine Kühlkörper sind relativ zueinander bewegbar angeordnet. Wenn das Maulteil geschlossen wird, wirkt eine Schließ- oder Klemmkraft (des Maulteils) auf die zumindest eine Metallelektrode und den zumindest einen Kühlkörper. Die Schließkraft bewirkt eine Relativbewegung der zumindest einen Metallelektrode und des zumindest einen Kühlkörpers zueinander. Durch die Relativbewegung kann die zumindest eine Metallelektrode von dem zumindest einen Kühlkörper (thermisch) getrennt werden. Beim Öffnen des Maulteils entfällt die Schließkraft. Das Entfallen der Schließkraft bewirkt wiederum eine entgegengesetzte Relativbewegung oder Öffnungsbewegung zwischen der zumindest einen Metallelektrode und dem zumindest einen Kühlkörper. Durch die Öffnungsbewegung werden die zumindest eine Metallelektrode und der zumindest eine Kühlkörper derart miteinander verbunden, dass sie thermisch gekoppelt sind bzw. in der wärmeleitenden Verbindung zueinanderstehen.

In anderen Worten ist ein chirurgisches Instrument, insbesondere HF-Instrument offenbart, mit:
- einem Maulteil mit zwei Schenkeln oder Branchen, die relativ zueinander zwischen einer Gewebe-Freigabestellung und einer Gewebe-Einspannstellung des Maulteils bewegbar sind;
- wenigstens einer, wahlweise mit einem elektrischen Strom beaufschlagbaren Elektrode, die an einem Schenkel auf einer dem anderen Schenkel zugewandten Seite gelagert ist; und
- einem Kühlkörper, der auf einer dem anderen Schenkel abgewandten Seite der wenigstens einen Elektrode angeordnet ist, sowie mit
- einer Vorspanneinrichtung, vorzugsweise Feder, die dafür vorgesehen und eingerichtet ist, die wenigstens eine Elektrode gegen eine thermische Kontaktfläche des Kühlkörpers für eine Wärmeübertragung vorzuspannen und deren Vorspannrichtung hin zum anderen Schenkel weist, sodass in der Gewebe-Einspannstellung die zumindest eine Elektrode von der thermischen Kontaktfläche entgegen einer Federvorspannung beabstandet ist und in der Gewebe-Freigabestellung die zumindest eine Elektrode und die thermische Kontaktfläche des Kühlkörper in wärmeleitender Anlage miteinander stehen.

Der Kern der vorliegenden Offenbarung besteht also darin, dass zumindest eine Metallelektrode und zumindest ein Kühlkörper eines chirurgischen Instruments (durch eine Relativbewegung) thermisch voneinander entkoppelt und wieder gekoppelt werden können. D.h. die Wärmeleitfähigkeit zwischen der zumindest einen Metallelektrode und dem zumindest einen Kühlkörper kann abhängig von dem Betriebszustand des chirurgischen Instruments variiert werden. Konkret ist der zumindest eine Kühlkörper an die zumindest eine Metallelektrode anschließbar- und auch abkoppelbar.

Das chirurgische Instrument gemäß der vorliegenden Offenbarung hat dabei die folgenden Vorteile. Die zumindest eine Metallelektrode und der zumindest eine Kühlkörper können je nach Betriebszustand thermisch entkoppelt und wieder gekoppelt werden. D.h. der zumindest eine Kühlkörper bzw. die Kühlmasse des zumindest einen Kühlkörpers kann derart groß ausgelegt werden, dass der zumindest eine Kühlkörper die zumindest eine Metallelektrode effektiv kühlen kann, wenn die zumindest eine Metallelektrode nicht im Einsatz ist. Gleichzeitig kann sichergestellt werden, dass die große Kühlmasse des Kühlkörpers die Metallelektrode nicht unnötig stark abkühlt, wenn die Metallelektrode zum Versiegeln von Gewebe erhitzt werden soll. Dadurch kann die Metallelektrode das Gewebe effektiv versiegeln. Gleichzeitig kann sich die Metallelektrode durch die große anschließbare Kühlmasse schnell abkühlen, wenn das chirurgische Instrument nicht im Einsatz ist. Somit können Schäden am umliegenden Gewebe durch (versehentlichen) Kontakt mit der (erhitzten) Metallelektrode verhindert oder zumindest gemindert werden. Ferner ist das Maulteil verschlossen, sodass keine Verunreinigungen eintreten können.

Vorteilhafte Weiterbildungen der vorliegenden Offenbarung sind Gegenstand der beigefügten Unteransprüche.

Vorzugsweise kann das Instrument ein elastisches Element, insbesondere eine Feder, aufweisen, die in dem geschlossenen Zustand durch die Schließkraft des Maulteils komprimiert ist. Bei einem Wegfall / Abfallen der Schließkraft kann eine Federkraft des elastischen Elements die Relativbewegung der zumindest einen Metallelektrode und des zumindest einen Kühlkörpers zueinander bewirken. D.h. der zumindest eine Kühlkörper und die zumindest eine Metallelektrode können mit zumindest einem elastischen Element vorgespannt sein. Das zumindest eine elastische Element kann insbesondere dann komprimiert werden, wenn das Maulteil geschlossen wird. Entfällt die Kraft, die die Schenkel des Maulteils zusammenpresst, können die zumindest eine Metallelektrode und der zumindest eine Kühlkörper durch die Federkraft des elastischen Elements relativ zueinander bewegt werden. Dadurch können die zumindest eine Metallelektrode und der zumindest eine Kühlkörper "automatisch" oder ohne weitere Betätigung durch einen Nutzer zumindest abschnittweise verbunden und dadurch thermisch gekoppelt werden, wenn das Maulteil geöffnet wird. Somit kann die Metallelektrode beim Öffnen des Maulteils effektiv gekühlt werden. Durch das elastische Element kann also die Kühlung zugeschaltet werden, ohne, dass der Nutzer dies manuell betätigen muss.

Durch die Relativbewegung der zumindest einen Metallelektrode und des zumindest einen Kühlkörpers zueinander kann zwischen den beiden Komponenten ein Luftspalt entstehen, der eine thermische Isolierung zwischen den beiden Komponenten bewirkt.

Bei dem zumindest einen elastischen Element kann es sich vorzugsweise um eine Schraubenfeder handeln. Schraubenfedern sind insbesondere kostengünstige Bauteile. Vorzugsweise kann die zumindest eine Feder derart angeordnet sein, dass die Federkraft senkrecht zu einer Längserstreckung der einzelnen Schenkel wirkt. Konkret kann die Federkraft also im Wesentlichen entgegen der Schließkraft des Maulteils wirken. Das elastische Element kann auch ein elastisches Kissen sein, das vorzugsweise aus einem elastischen Material wie beispielsweise einem elastischen Schaumstoff gefertigt sein kann.

In einer bevorzugten Ausführungsform kann jeder Schenkel des Maulteils zwei elastische Elemente aufweisen, die eine Metallelektrode relativ zu einem Kühlkörper bewegen. Selbstverständlich kann das Maulteil aber auch ein elastisches Element, drei oder vier oder eine beliebige Anzahl an elastischen Elementen aufweisen.

Nach einem vorteilhaften Aspekt der vorliegenden Offenbarung kann das elastische Element die zumindest eine Metallelektrode und den zumindest einen Kühlkörper zumindest abschnittsweise auseinanderdrücken. Das elastische Element ist in dem geschlossenen Zustand derart komprimiert oder vorgespannt, dass sich das elastische Element bei einem Übergang von dem geschlossenen Zustand in den geöffneten Zustand entspannt und die zumindest eine Metallelektrode und den zumindest einen Kühlkörper relativ zueinander bewegt. Die zumindest eine Metallelektrode und der zumindest eine Kühlkörper können aber derart ausgestaltet sein, dass zumindest Abschnitte der beiden Komponenten trotz der Relativbewegung, die die Komponenten auseinanderdrückt, aneinander anliegen und somit thermisch gekoppelt sind. Durch die Relativbewegung zwischen der zumindest einen Metallelektrode und dem zumindest einen Kühlkörper, die die abschnittsweise thermische Kopplung ermöglicht, kann der zumindest eine Kühlkörper an die zumindest eine Metallelektrode angeschlossen werden und dadurch die zumindest eine Metallelektrode kühlen.

Vorzugsweise können die zumindest eine Metallelektrode und der zumindest eine Kühlkörper relativ zueinander in einer Bewegungsrichtung bewegbar sein, die sich senkrecht zu einer Längserstreckung der Schenkel erstreckt. Das elastische Element kann die Relativbewegung der zumindest einen Metallelektrode und des zumindest einen Kühlkörpers bedingen. Die Richtung der Relativbewegung kann also in Richtung der Federkraft bzw. in Schließrichtung wirken.

Nach einem weiteren vorteilhaften Aspekt der vorliegenden Offenbarung können die zumindest eine Metallelektrode und der zumindest eine Kühlkörper jeweils einen Hinterschnitt aufweisen. Die jeweiligen Hinterschnitte können in dem geschlossenen Zustand voneinander beabstandet sein und in dem geöffneten Zustand aneinander anliegen. Wenn die Hinterschnitte aneinander anliegen, dann können sie zumindest abschnittsweise eine Auflagefläche zwischen der zumindest einen Metallelektrode und dem zumindest einen Kühlkörper bilden. Über die Auflagefläche kann thermische Energie von der zumindest einen Metallelektrode auf den zumindest einen Kühlkörper übergehen. Somit können die zumindest eine Metallelektrode und der zumindest eine Kühlkörper im geöffneten Zustand thermisch miteinander gekoppelt sein und der zumindest eine Kühlkörper die zumindest eine Metallelektrode kühlen.

Vorzugsweise kann der Hinterschnitt die Relativbewegung zwischen der zumindest einen Metallelektrode und dem zumindest einen Kühlkörper in Bewegungsrichtung begrenzen. Somit kann der Hinterschnitt verhindern, dass die zumindest eine Metallelektrode und der zumindest eine Kühlkörper durch die Federkraft vollständig getrennt oder separiert werden. Vielmehr kann durch die Auflagefläche des Hinterschnitts die thermische Kopplung zwischen der zumindest einen Metallelektrode und dem zumindest einen Kühlkörper realisiert werden. Über die Auflagefläche kann die zumindest eine Metallelektrode durch den zumindest einen Kühlkörper abgekühlt werden.

Nach einem weiteren vorteilhaften Aspekt der vorliegenden Offenbarung kann die zumindest eine Metallelektrode einen Elektrodenkörper mit der Kontaktfläche und eine erste Zahnung mit einer Anzahl an ersten Zähnen aufweisen. Die ersten Zähne können sich dabei insbesondere von dem Elektrodenkörper aus in eine der Kontaktfläche abgewandten Seite erstrecken. Die zumindest eine Metallelektrode kann also einen Grundkörper aufweisen, von dem aus sich die ersten Zähne erstrecken. Die ersten Zähne können sich insbesondere in Richtung des zumindest einen Kühlkörpers erstrecken. Dadurch kann eine Verzahnungsgeometrie bereitgestellt werden, die dafür vorbereitet ist, mit einer entsprechenden Verzahnungsgeometrie des zumindest einen Kühlkörpers (zueinander beweglich) verbunden zu werden.

Vorzugsweise können die ersten Zähne der ersten Zahnung jeweils einen ersten Grundabschnitt, der sich vorzugsweise senkrecht zu dem Elektrodenkörper erstreckt, und einen ersten Kopfabschnitt aufweisen, wobei sich der erste Kopfabschnitt von dem ersten Grundabschnitt aus pilzförmig parallel zu dem Elektrodenkörper aufweitet. D.h. der erste Grundabschnitt kann sich senkrecht zu einer Längserstreckung des Kühlkörpers erstrecken bzw. senkrecht aus dem Kühlkörper hervorstehen. Der erste Kopfabschnitt kann sich dabei wiederum senkrecht zu dem ersten Grundabschnitt erstrecken. Somit kann sich der erste Kopfabschnitt in eine Längserstreckung der Schenkel erstrecken.

Nach einem weiteren vorteilhaften Aspekt der vorliegenden Offenbarung kann der zumindest eine Kühlkörper einen Grundkörper und eine zweite Zahnung mit einer Anzahl an zweiten Zähnen aufweisen. Die zweiten Zähne können sich von dem Grundkörper aus in Richtung der zumindest einen Metallelektrode erstrecken. Der Grundkörper des zumindest einen Kühlkörpers kann dabei eine Verlängerung der Schenkel bzw. einer Verbindung zwischen den Griffen und dem Maulteil sein. Die zweiten Zähne können sich senkrecht von dem Grundkörper aus in Richtung Metallelektrode erstrecken. Somit kann eine Verzahnungsgeometrie bereitgestellt werden, die dafür vorbereitet ist, mit der entsprechenden Verzahnungsgeometrie der Metallelektrode (beweglich) verbunden zu werden.

Vorzugsweise können die zweiten Zähne jeweils einen zweiten Grundabschnitt und einen zweiten Kopfabschnitt aufweisen. Der zweite Grundabschnitt kann vorzugsweise senkrecht aus dem Grundkörper hervorstehen. Der zweite Kopfabschnitt kann sich von dem zweiten Grundabschnitt aus pilzförmig parallel zu dem Grundkörper nach außen aufweiten. Der zweite Grundabschnitt kann also gerade in Richtung der Metallelektrode aus dem Grundkörper des Kühlkörpers vorstehen. Eine Längserstreckung des zweiten Kopfabschnitts kann sich somit parallel zu dem Grundkörper des Kühlkörpers bzw. entlang einer Längserstreckung der Schenkel erstrecken. Die ersten Kopfabschnitte und die zweiten Kopfabschnitte bilden zusammen den Hinterschnitt bzw. die Hinterschnitte.

Nach einem weiteren vorteilhaften Aspekt der vorliegenden Offenbarung können die ersten Zähne und die zweiten Zähne jeweils abwechselnd ineinandergreifen. D.h. die ersten Zähne der Metallelektrode können jeweils neben den zweiten Zähnen des Kühlkörpers angeordnet sein. Somit kann die Auflagefläche zwischen der zumindest einen Metallelektrode und dem zumindest einen Kühlkörper im geöffneten Zustand vergrößert werden.

Vorzugsweise können die ersten Zähne und die zweiten derart ineinandergreifen, dass die jeweiligen pilzförmigen Kopfabschnitte im geöffneten Zustand aneinander anliegen und den Hinterschnitt bzw. die mehreren (parallelen) Hinterschnitte bilden. D.h. im geöffneten Zustand können die pilzförmigen Kopfabschnitte die gemeinsame Auflagefläche für die thermische Kopplung bilden. Im geschlossenen Zustand dagegen können die pilzförmigen Kopfabschnitte derart voneinander beabstandet sein, dass die zumindest einen Metallelektrode und der zumindest eine Kühlkörper thermisch voneinander entkoppelt sind. Zwischen den pilzförmigen Kopfabschnitten kann also der Luftspalt ausgebildet sein, der die thermische Isolierung bewirkt.

In anderen Worten können die jeweiligen Schenkel eine Verzahnung aufweisen, die in einer Aussparung bzw. einer entsprechenden Verzahnung der jeweils anderen Komponente geführt ist und den Hinterschnitt bildet.

Nach einem weiteren vorteilhaften Aspekt der vorliegenden Offenbarung kann der erste Kopfabschnitt der zumindest einen Metallelektrode von der Metallelektrode aus in Bewegungsrichtung gesehen hinter dem zweiten Kopfabschnitt des zumindest einen Kühlkörpers angeordnet sein. Somit kann der Hinterschnitt zwischen der zumindest einen Metallelektrode und dem zumindest einen Kühlkörper ausgebildet werden.

Vorzugsweise können die jeweiligen Kopfabschnitte bei einer Bewegung der Schenkel von dem geöffneten Zustand in den geschlossenen Zustand an den Grundabschnitten der entsprechenden Zähne abgleiten bzw. durch die Grundabschnitte der entsprechenden Zähne geführt sein. Somit können die zumindest eine Metallelektrode und der zumindest eine Kühlkörper relativ zueinander bewegbar sein.

Nach einem weiteren vorteilhaften Aspekt der vorliegenden Offenbarung kann zumindest ein Abstandhalter, der vorzugsweise dreieckig ausgebildet ist, an der zumindest einen Metallelektrode und/oder an dem zumindest einen Kühlkörper angeordnet sein. Durch den zumindest einen Abstandhalter kann ein Abstand zwischen der zumindest einen Metallelektrode und dem zumindest einen Kühlkörper vergrößert werden. Ferner kann dadurch sichergestellt werden, dass im geschlossenen Zustand kein direkter Kontakt zwischen der zumindest einen Metallelektrode und dem zumindest einen Kühlkörper hergestellt ist. Über den direkten Kontakt könnte thermische Energie übertragen werden, was im geschlossenen Zustand nicht gewünscht ist. Der zumindest eine Abstandhalter kann dabei auch prismenförmig ausgebildet sein.

Nach einem weiteren vorteilhaften Aspekt der vorliegenden Offenbarung kann die zumindest eine Metallelektrode derart (beweglich oder schwenkbar) auf einer Wippe gelagert sein, dass die zumindest eine Metallelektrode bei einer Bewegung der Schenkel von dem geöffneten Zustand in den geschlossenen Zustand von dem zumindest einen Kühlkörper weggeschwenkt wird. D.h. die Metallelektrode kann drehbar auf der Wippe gelagert / angeordnet sein und um einen Drehpunkt der Wippe geschwenkt werden. In dem geschlossenen Zustand kann die Metallelektrode derart ausgerichtet sein, dass die Kontaktfläche das zu versiegelnde Gewebe berührt. In dem geöffneten Zustand dagegen, kann die Metallelektrode derart ausgerichtet sein, dass sie von dem Kühlkörper gekühlt wird. Die Wippe kann somit sowohl eine optimale Ausrichtung der Kontaktfläche bezüglich des Gewebes als auch eine koppelbare Verbindung zwischen der Metallelektrode und dem Kühlkörper bewirken.

Vorzugsweise kann die Federkraft des zumindest einen elastischen Elements die zumindest eine Metallelektrode in dem geöffneten Zustand an den zumindest einen Kühlkörper anpressen. Das zumindest eine elastische Element kann bezüglich der Wippe derart angeordnet sein, dass das zumindest eine elastische Element die zumindest eine Metallelektrode an den zumindest einen Kühlkörper anpresst. D.h. das zumindest eine elastische Element kann auf der einen Seite der Wippe angeordnet sein, wobei die Federkraft des elastischen Elements derart auf die Metallelektrode wirkt, dass die Metallelektrode um den Drehpunkt der Wippe geschwenkt wird und auf der anderen Seite der Wippe an den Kühlkörper angedrückt oder angepresst wird. Dadurch kann die Metallelektrode in dem geöffneten Zustand des Maulteils, in dem die Metallelektrode frei um die Wippe drehbar ist, in Kontakt mit dem Kühlkörper stehen und von diesem gekühlt werden.

Die Kontaktfläche zwischen der zumindest einen Metallelektrode und dem zumindest einen Kühlkörper kann durch eine Verzahnung oder eine raue (Kontakt-)Oberfläche vergrößert werden, um eine effizientere Wärmeübertragung zu ermöglichen.

In dem geschlossenen Zustand kann das elastische Element durch die Schließkraft des Maulteils komprimiert sein. D.h. die Federkraft kann im Wesentlichen entgegen der Schließkraft des Maulteils wirken.

Das Maulteil kann entweder zwei Metallelektroden aufweisen (bipolare HF-Technik) oder lediglich eine (Metall-)Elektrode (monopolare HF-Technik). Bei der monopolaren HF-Technik kann die zweite (Ableit-)Elektrode an dem Körper des Patienten befestigt sein.

Bei dem chirurgischen Instrument kann es sich insbesondere um ein elektrochirurgisches Instrument für offenchirurgische Eingriffe handeln. Das chirurgische Instrument kann aber auch für minimal invasive chirurgische Eingriffe geeignet sein.

### Kurzbeschreibung der Figuren

Fig. 1 zeigt eine schematische Darstellung eines chirurgischen Instruments gemäß einer ersten Ausführungsform der vorliegenden Offenbarung in einem geöffneten Zustand;
Fig. 2 zeigt eine Detailansicht eines Maulteils des chirurgischen Instruments gemäß der ersten Ausführungsform der vorliegenden Offenbarung in dem geöffneten Zustand;
Fig. 3 zeigt eine Detailansicht eines Maulteils des chirurgischen Instruments gemäß der ersten Ausführungsform der vorliegenden Offenbarung in einem geschlossenen Zustand;
Fig. 4 zeigt einen Längsschnitt durch ein Maulteil des chirurgischen Instruments gemäß einer zweiten Ausführungsform der vorliegenden Offenbarung in einem geschlossenen Zustand; und
Fig. 5 zeigt einen Längsschnitt durch ein Maulteil des chirurgischen Instruments gemäß der zweiten Ausführungsform der vorliegenden Offenbarung in einem geöffneten Zustand.

### Detaillierte Beschreibung der Figuren

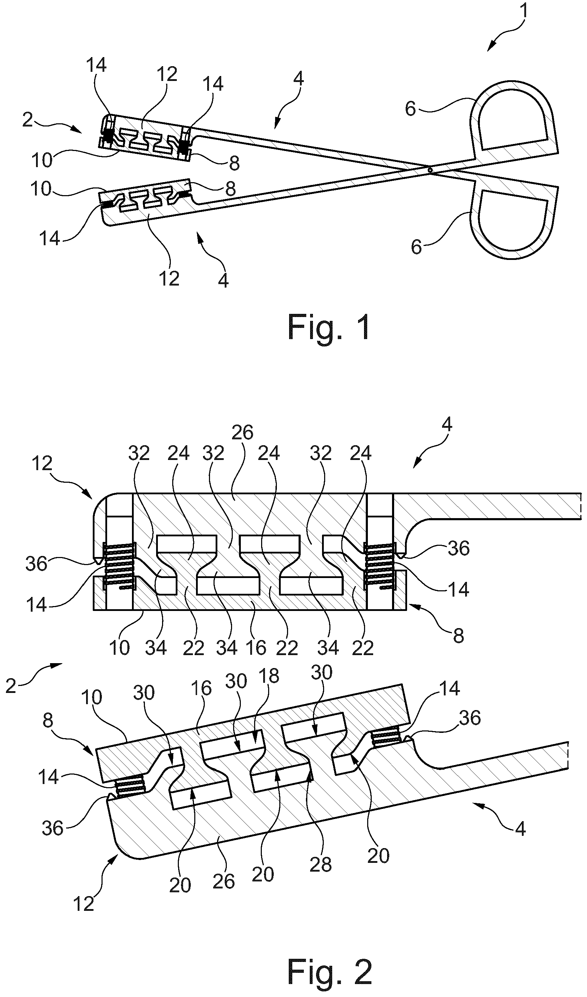

Fig. 1 zeigt ein chirurgisches Instrument 1 mit einem Maulteil 2 mit zwei Schenkeln 4. Die zwei Schenkel 4 sind relativ zueinander zwischen einem geöffneten Zustand oder einem Freigabezustand und einem geschlossenen Zustand oder einem Klemmzustand des Maulteils 2 bewegbar, wobei die Fig. 1 das chirurgische Instrument 1 in dem geöffneten Zustand zeigt. Das chirurgische Instrument 1 weist ferner zwei (Scheren-)Griffe 6 auf, durch die ein Nutzer die Schenkel 4 bewegen kann. Jeder Schenkel 4 des Maulteils 2 hat eine Metallelektrode 8 mit einer Kontaktfläche 10 zum Kontaktieren von Gewebe und einen Kühlkörper 12, der auf einer von der Kontaktfläche 10 abgewandten Seite angeordnet ist. Der Kühlkörper 12 ist mit dem jeweiligen Griff 6 verbunden. Jeder Schenkel 4 weist zwei elastische Elemente bzw. Federn 14 auf, die den Kühlkörper 12 und die Metallelektrode 8 auseinander drücken. In dem geöffneten Zustand oder Freigabezustand sind die einzelnen Schenkel und damit auch die einzelnen Kontaktflächen 10 des Instruments 1 voneinander beabstandet. D.h. die beiden Kontaktflächen 10 liegen nicht aneinander an.

Fig. 2 zeigt eine Detailansicht eines der Schenkel 4 des geöffneten Maulteils 2. Die Federn 14 sind dabei jeweils in einer entspannten Grundstellung ausgedehnt. Die Metallelektroden 8 weisen jeweils einen Elektrodenkörper 16 mit der Kontaktfläche 10 und eine erste Zahnung 18 auf. Die erste Zahnung 18 hat eine Anzahl an ersten Zähnen 20, die sich von dem Elektrodenkörper 16 in die der Kontaktfläche 10 abgewandten Seite erstrecken. Die Zähne 20 weisen jeweils einen ersten Grundabschnitt 22 und einen ersten Kopfabschnitt 24 auf. Der erste Grundabschnitt 22 erstreckt sich senkrecht zu dem Elektrodenkörper 16 und der erste Kopfabschnitt 24 weitet sich von dem ersten Grundabschnitt 22 aus pilzförmig nach außen auf. D.h. der erste Kopfabschnitt 24 erstreckt sich zumindest abschnittsweise parallel zu dem Elektrodenkörper 16.

Die Kühlkörper 12 weisen jeweils einen Grundkörper 26 und eine zweite Zahnung 28 mit einer Anzahl an zweiten Zähnen 30 auf. Die zweiten Zähne 30 erstrecken sich von dem Grundkörper 26 aus in Richtung hin zu der zumindest einen Metallelektrode 8. D.h. die zweiten Zähne 30 erstrecken sich von dem Grundkörper 26 zu der zumindest einen Metallelektrode 8. Die zweiten Zähne 30 haben jeweils einen zweiten Grundabschnitt 32, der sich vorzugsweise senkrecht zu dem Grundkörper 26 erstreckt. Ferner haben die zweiten Zähne 30 einen zweiten Kopfabschnitt 34, der sich von dem zweiten Grundabschnitt 32 aus pilzförmig nach außen aufweitet. D.h. der zweite Kopfabschnitt 34 erstreckt sich zumindest abschnittsweise parallel zu dem Grundkörper 26.

Die ersten Zähne 20 der Metallelektrode 8 und die zweiten Zähne 30 des Kühlkörpers 12 sind jeweils (abwechselnd) nebeneinander angeordnet und greifen ineinander ein. Die jeweiligen Zahnungen 18, 28 der Metallelektrode 8 und des Kühlkörpers 12 sind dabei derart angeordnet, dass die Metallelektrode 8 und der Kühlkörper 12 relativ zu einander bewegbar sind. Die Metallelektrode 8 und der Kühlkörper 12 sind dabei vorzugsweise in einer Bewegungsrichtung senkrecht zur Längserstreckung der Schenkel 4 zueinander bewegbar. Bei der Relativbewegung gleitet der erste pilzförmige Kopfabschnitt 24 der Metallelektrode 8 an dem zweiten Grundabschnitt 32 des Kühlkörpers 12 entlang. Entsprechend gleitet der zweite pilzförmige Kopfabschnitt 34 an dem ersten Grundabschnitt 22 entlang. Die Relativbewegung wird durch die beiden Kopfabschnitte 24, 34 begrenzt, die im geöffneten Zustand des Maulteils 2 aneinander anliegen und den Hinterschnitt ausbilden.

Der erste pilzförmige Kopfabschnitt 24 und der zweite pilzförmige Kopfabschnitt 34 greifen ineinander ein bilden zusammen einen Hinterschnitt aus, der die Relativbewegung der zumindest einen Metallelektrode 8 und des zumindest einen Kühlkörpers 12 in Bewegungsrichtung begrenzt. Dadurch, dass die jeweiligen Kopfabschnitte 24 und 34 aneinander anliegen, weisen die jeweiligen Zähne 20 und 30 eine große Auflagefläche zueinander auf. Durch die große Auflagefläche der Zähne 20, 30 kann thermische Energie von der Metallelektrode 8 zu dem Kühlkörper 12 transportiert werden. D.h. die Metallelektrode 8 und der Kühlkörper 12 sind im geöffneten Zustand thermisch gekoppelt. In diesem Zustand kann der Kühlkörper 12 thermische Energie von der Metallelektrode 8 aufnehmen und die Metallelektrode 8 somit abkühlen. Den Metallelektroden 8 wird in dem geöffneten Zustand also thermische Energie durch die entsprechenden, anliegenden Kühlkörper 12 entzogen.

Das chirurgische Instrument 1 ist insbesondere ein Hochfrequenz-Instrument zum Versiegeln von Gewebe. Zum Versiegeln von Gewebe werden die beiden Schenkel 4 des Maulteils 2 durch eine Schließ- oder Klemmkraft zusammengedrückt und geschlossen. In dem geschlossenen Zustand oder Klemmzustand wird das zu versiegelnde Gewebe dabei zwischen den zwei Kontaktflächen 10 eingeklemmt und mit hochfrequentem Strom durchflossen / durchströmt. Dadurch erhitzt sich das Gewebe, das zwischen den zwei Kontaktflächen 10 eingeklemmt ist. Da zum Versiegeln Wasser aus dem Gewebe verdampfen soll, wird eine Oberflächentemperatur von (mindestens) 100° C an den Kontaktflächen 10 benötigt. Eine thermische Kopplung der Metallelektroden 8 mit den Kühlkörpern 12 würde den Metallelektroden 8 viel thermische Energie entziehen und ein Erhitzen der Metallelektroden 8 verlängern. Der Versiegelungsprozess wäre somit ineffizient.

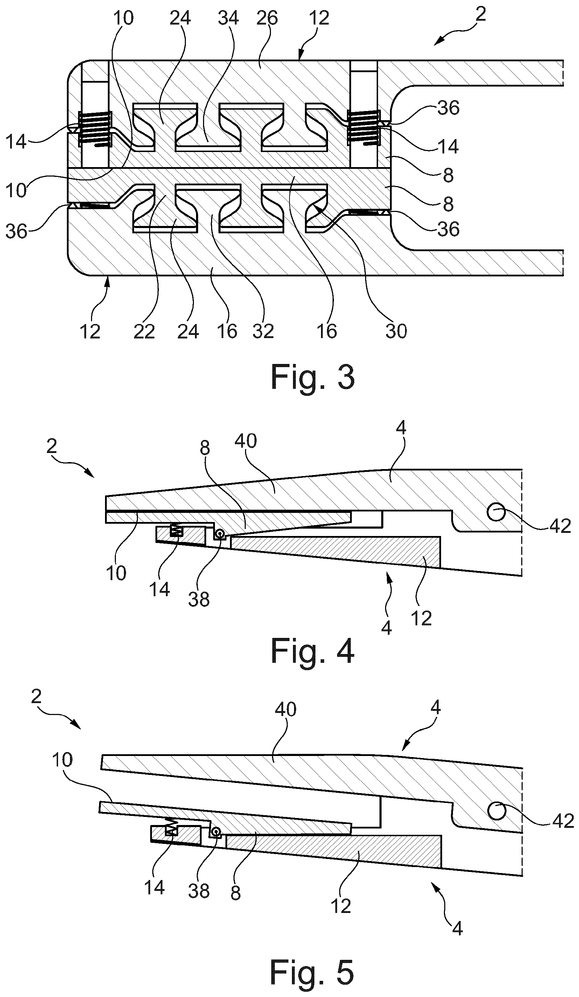

Fig. 3 zeigt eine Detailansicht eines der Schenkel 4 des geschlossenen Maulteils 2. Wenn das Maulteil 2 geschlossen ist, dann liegen die beiden Kontaktflächen 10 der jeweiligen Schenkel 4 aufeinander auf und werden durch eine Schließkraft zusammengepresst. Die beiden Metallelektroden 8 sollen heiß sein, damit Gewebe, das zwischen den beiden Metallelektroden 8 angeordnet ist, durch Einbringung von thermischer Energie versiegelt werden kann. Deshalb sollten die Metallelektrode 8 und der Kühlkörper 12 in dem geschlossenen Zustand thermisch entkoppelt sein.

Die Schließkraft des Maulteils komprimiert die Federn 14 und bewegt die Metallelektrode 8 in Richtung des Kühlkörpers 12. Bei der Schließbewegung gleiten die pilzförmigen Kopfabschnitte 24, 34 an den Grundabschnitten 22, 32 der jeweils anliegenden Zähne 20, 30 entlang. In dem geschlossenen Zustand liegen die pilzförmigen Kopfabschnitte 24, 34 nicht mehr aneinander an. Dadurch verringert sich die Auflagefläche zwischen der Metallelektrode 8 und dem Kühlkörper 12. Somit ist weniger Fläche für einen Wärmeaustausch zwischen der Metallelektrode 8 und dem Kühlkörper 12 vorhanden. D.h. ein Luftspalt ist zwischen den jeweiligen Kopfabschnitten 24, 34 vorhanden und isoliert die Metallelektrode 8 von dem Kühlkörper. Die Metallelektrode 8 kann sich somit aufheizen, ohne dass die thermische Energie von der großen (Kühl-)Masse des Kühlkörpers 12 entzogen wird. Der Kühlkörper 12 weist ferner zwei dreieckige Abstandshalter 36 auf, die auf der Metallelektrode 8 aufliegen und den Abstand zwischen der Metallelektrode 8 und dem Kühlkörper 12 vergrößern. Einer der Abstandshalter 36 ist dabei an einem distalen Endabschnitt des Maulteils angeordnet und der andere der Abstandshalter 36 ist an einem proximalen Endabschnitt der Metallelektrode 8 angeordnet. Die Abstandshalter 36 sind vorzugsweise aus einem thermisch isolierenden Material, wie beispielsweise Keramik gefertigt.

Fig. 4 zeigt das Maulteil 2 nach einer weiteren Ausführungsform der vorliegenden Offenbarung in dem geschlossenen Zustand oder Klemmzustand. Die Metallelektrode 8 ist auf einer Wippe 38 (schwenkbar) gelagert. Wenn das Maulteil in dem geschlossenen Zustand oder Klemmzustand ist, dann liegt die Metallelektrode 8 flächig an dem anderen Schenkel 40 an, der vorzugsweise eine Neutralelektrode ist. In dem Klemmzustand ist die Feder 14 durch die Schließ- oder Klemmkraft des Maulteils 2 komprimiert. Die Metallelektrode 8 hat lediglich einen minimalen Kontakt zu dem Kühlkörper 12. Somit kann die Metallelektrode 8 effektiv zum Versiegeln von Gewebe erhitzt werden. Der weitere Schenkel 40 ist um ein Scharnier 42 schwenkbar und übt die Schließ- oder Klemmkraft auf die Metallelektrode 8 aus.

Fig. 5 zeigt ein Maulteil 2 nach der weiteren Ausführungsform in dem geöffneten oder Freigabezustand. Die Schenkel 4 sind voneinander beabstandet. D.h. der Schenkel 40 ist von der Metallelektrode 8 weggeschwenkt. Dadurch entfällt die Schließ- oder Klemmkraft auf die Metallelektrode 8. Die Feder 14 entspannt sich in ihre Grundstellung und die Federkraft schwenkt die Metallelektrode 8 um einen Drehpunkt der Wippe 38. Somit wird die Metallelektrode 8 durch die Federkraft an den Kühlkörper 12 angepresst. Die Metallelektrode 8 kann somit in dem geöffneten Zustand durch den Kühlkörper 12 abgekühlt werden. Eine Kontaktfläche oder Auflagefläche zwischen der Metallelektrode 8 und dem Kühlkörper 12 kann auch eine Verzahnung oder aufgeraute Oberfläche aufweisen, um die Wärmeübertragung zu verbessern.

Selbstverständlich kann auch das Maulteil 2 der zweiten Ausführungsform zwei Metallelektroden 8 und damit zwei Kühlkörper 12 aufweisen. Das Maulteil 2 kann entweder zwei Metallelektroden aufweisen (bipolare HF-Technik) oder lediglich eine Elektrode (monopolare HF-Technik).

### Bezugszeichenliste

- 1: Instrument
- 2: Maulteil
- 4: Schenkel
- 6: Griffe
- 8: Metallelektrode
- 10: Kontaktfläche
- 12: Kühlkörper
- 14: elastisches Element / Feder
- 16: Elektrodenkörper
- 18: erste Zahnung
- 20: erste Zähne
- 22: erster Grundabschnitt
- 24: erster Kopfabschnitt
- 26: Grundkörper
- 28: zweite Zahnung
- 30: zweite Zähne
- 32: zweiter Grundabschnitt
- 34: zweiter Kopfabschnitt
- 36: Abstandhalter
- 38: Wippe
- 40: weiterer Schenkel
- 42: Scharnier

## Patentansprüche

1. Chirurgisches Instrument (1), insbesondere HF-Instrument, mit einem Maulteil (2) mit zwei Schenkeln (4), die relativ zueinander zwischen einem geöffneten Zustand und einem geschlossenen Zustand des Maulteils (2) bewegbar sind,
wobei das Maulteil (2) zumindest eine Metallelektrode (8) mit einer Kontaktfläche (10) zum Kontaktieren von Gewebe und einen von der Kontaktfläche (10) abgewandten Kühlkörper (12) aufweist,
**dadurch gekennzeichnet, dass**
in dem geschlossenen Zustand die zumindest eine Metallelektrode (8) und der zumindest eine Kühlkörper (12) voneinander beabstandet und thermisch getrennt sind und in dem geöffneten Zustand die zumindest eine Metallelektrode (8) und der zumindest eine Kühlkörper (12) in einer wärmeleitenden Verbindung miteinander stehen.

2. Instrument (1) nach Anspruch 1, weiterhin aufweisend zumindest ein elastisches Element (14), insbesondere eine Feder, die in dem geschlossenen Zustand durch eine Schließkraft des Maulteils (2) komprimiert ist und bei einem Wegfall der Schließkraft die zumindest eine Metallelektrode (8) und den zumindest einen Kühlkörper (12) relativ zueinander bewegt.

3. Instrument (1) nach Anspruch 2, wobei das zumindest eine elastische Element (14) die zumindest eine Metallelektrode (8) und den zumindest einen Kühlkörper (12) auseinanderdrückt.

4. Instrument (1) nach einem der Ansprüche 1 bis 3, wobei zumindest eine Metallelektrode (8) und der zumindest eine Kühlkörper (12) relativ zueinander in einer Bewegungsrichtung senkrecht zu einer Längserstreckung der Schenkel (4) bewegbar sind.

5. Instrument (1) nach einem der Ansprüche 1 bis 4, wobei die zumindest eine Metallelektrode (8) und der zumindest eine Kühlkörper (12) jeweils zumindest einen Hinterschnitt aufweisen, wobei die jeweiligen Hinterschnitte in dem geschlossenen Zustand voneinander beabstandet sind und in dem geöffneten Zustand aneinander anliegen.

6. Instrument (1) nach Anspruch 5, wobei der Hinterschnitt die Relativbewegung zwischen der zumindest einen Metallelektrode (8) und dem zumindest einen Kühlkörper (12) in der Bewegungsrichtung begrenzt.

7. Instrument (1) nach einem der Ansprüche 1 bis 6, wobei die zumindest eine Metallelektrode (8) einen Elektrodenkörper (16) mit der Kontaktfläche (10) und eine erste Zahnung (18) mit einer Anzahl an ersten Zähnen (20) aufweist, die sich von dem Elektrodenkörper (16) aus in die der Kontaktfläche (10) abgewandten Seite erstrecken.

8. Instrument (1) nach Anspruch 7, wobei die ersten Zähne (20) der ersten Zahnung (18) jeweils einen ersten Grundabschnitt (22), der sich vorzugsweise senkrecht zu dem Elektrodenkörper (16) erstreckt, und einen ersten Kopfabschnitt (24) aufweisen, wobei sich der erste Kopfabschnitt (24) von dem ersten Grundabschnitt (22) aus pilzförmig parallel zu dem Elektrodenkörper (16) aufweitet.

9. Instrument (1) nach einem der Ansprüche 1 bis 8, wobei der zumindest eine Kühlkörper (12) einen Grundkörper (26) und eine zweite Zahnung (28) mit einer Anzahl an zweiten Zähnen (30) aufweist, die sich von dem Grundkörper (26) aus in Richtung der zumindest einen Metallelektrode (8) erstrecken.

10. Instrument (1) nach Anspruch 9, wobei die zweiten Zähne (30) jeweils einen zweiten Grundabschnitt (32), der sich vorzugsweise senkrecht zu dem Grundkörper (26) erstreckt, und einen zweiten Kopfabschnitt (34) aufweisen, wobei sich der zweite Kopfabschnitt (34) von dem zweiten Grundabschnitt (32) aus pilzförmig parallel zu dem Grundkörper (26) aufweitet.

11. Instrument (1) nach Anspruch 9 oder 10, wobei die ersten Zähne (20) und die zweiten Zähne (30) jeweils abwechselnd ineinandergreifen.

12. Instrument (1) nach Anspruch 10 oder 11, wobei die ersten Zähne (20) und die zweiten Zähne (30) derart ineinandergreifen, dass die jeweiligen pilzförmigen Kopfabschnitte (24, 34) in dem geöffneten Zustand aneinander anliegen und den Hinterschnitt bilden und in dem geschlossenen Zustand voneinander beabstandet sind.

13. Instrument (1) nach einem der Ansprüche 1 bis 12, wobei zumindest ein Abstandhalter (36), der vorzugsweise dreieckig ausgebildet ist, an der zumindest einen Metallelektrode (8) und/oder an dem zumindest einen Kühlkörper (12) angeordnet ist.

14. Instrument (1) nach einem der Ansprüche 1 bis 4, wobei die zumindest eine Metallelektrode (8) derart auf einer Wippe (38) gelagert ist, dass die zumindest eine Metallelektrode (8) bei einer Bewegung der Schenkel (4) von dem geöffneten Zustand in den geschlossenen Zustand von dem zumindest einen Kühlkörper (12) weggeschwenkt wird.

15. Instrument (1) nach Anspruch 14, wobei eine Federkraft des elastischen Elements (14) die zumindest eine Metallelektrode (8) im geöffneten Zustand um die Wippe (38) umschwenkt und an den zumindest einen Kühlkörper (12) anpresst.

## Claims

1. A surgical instrument (1), in particular an HF instrument, having a jaw part (2) with two legs (4) which can be moved relative to each other between an open state and a closed state of the jaw part (2),
wherein the jaw part (2) has at least one metal electrode (8) with a contact surface (10) for contacting tissue and a heatsink (12) facing away from the contact surface (10),
**characterized in that**
in the closed state, the at least one metal electrode (8) and the at least one heatsink (12) are spaced apart from each other and thermally separated, and in the open state, the at least one metal electrode (8) and the at least one heatsink (12) are in a thermally conductive connection with each other.

2. The instrument (1) according to claim 1, further comprising at least one elastic element (14), in particular a spring, which is compressed in the closed state by a closing force of the jaw part (2) and moves the at least one metal electrode (8) and the at least one heatsink (12) relative to each other when the closing force is removed.

3. The instrument (1) according to claim 2, wherein the at least one elastic element (14) pushes the at least one metal electrode (8) and the at least one heatsink (12) apart.

4. The instrument (1) according to one of claims 1 to 3, wherein the at least one metal electrode (8) and the at least one heatsink (12) are movable relative to each other in a direction of movement perpendicular to a longitudinal extension of the leg (4).

5. The instrument (1) according to one of claims 1 to 4, wherein the at least one metal electrode (8) and the at least one heatsink (12) each have at least one undercut, wherein the respective undercuts are spaced apart from each other in the closed state and abut against each other in the open state.

6. The instrument (1) according to claim 5, wherein the undercut limits the relative movement between the at least one metal electrode (8) and the at least one heatsink (12) in the direction of movement.

7. The instrument (1) according to one of claims 1 to 6, wherein the at least one metal electrode (8) comprises an electrode body (16) with the contact surface (10) and a first toothing (18) with a number of first teeth (20) extending from the electrode body (16) into the side facing away from the contact surface (10).

8. The instrument (1) according to claim 7, wherein the first teeth (20) of the first toothing (18) each have a first base portion (22), which preferably extends perpendicular to the electrode body (16), and have a first head portion (24), wherein the first head portion (24) widens from the first base portion (22) in a mushroom shape parallel to the electrode body (16).

9. The instrument (1) according to one of claims 1 to 8, wherein the at least one heatsink (12) comprises a base body (26) and a second toothing (28) with a number of second teeth (30) extending from the base body (26) toward the at least one metal electrode (8).

10. The instrument (1) according to claim 9, wherein the second teeth (30) each comprise a second base portion (32), which preferably extends perpendicular to the base body (26), and have a second head portion (34), wherein the second head portion (34) widens from the second base portion (32) in a mushroom shape parallel to the base body (26).

11. The instrument (1) according to claim 9 or 10, wherein the first teeth (20) and the second teeth (30) each alternately engage with each other.

12. The instrument (1) according to claim 10 or 11, wherein the first teeth (20) and the second teeth (30) interlock in such a way that the respective mushroom-shaped head portions (24, 34) abut against each other in the open state and form the undercut and are spaced apart from each other in the closed state.

13. The instrument (1) according to one of claims 1 to 12, wherein at least one spacer (36), which is preferably triangular in shape, is arranged on the at least one metal electrode (8) and/or on the at least one heatsink (12).

14. The instrument (1) according to one of claims 1 to 4, wherein the at least one metal electrode (8) is mounted on a rocker (38) such that the at least one metal electrode (8) is pivoted away from the at least one heatsink (12) during a movement of the leg (4) from the open state into the closed state.

15. The instrument (1) according to claim 14, wherein a spring force of the elastic element (14) swivels the at least one metal electrode (8) in the open state around the rocker (38) and presses it against the at least one heatsink (12).

## Revendications

1. Instrument chirurgical (1), en particulier instrument HF, avec une partie mâchoire (2) à deux branches (4) qui sont mobiles l'une par rapport à l'autre entre un état ouvert et un état fermé de la partie mâchoire (2),
dans lequel la partie mâchoire (2) présente au moins une électrode métallique (8) avec une surface de contact (10) pour la mise en contact de tissu et un corps de refroidissement (12) éloigné de la surface de contact (10),
**caractérisé en ce que**
à l'état fermé, la au moins une électrode métallique (8) et le au moins un corps de refroidissement (12) sont espacés et séparés thermiquement l'un de l'autre et à l'état ouvert, la au moins une électrode métallique (8) et le au moins un corps de refroidissement (12) sont en liaison thermoconductrice l'un avec l'autre.

2. Instrument (1) selon la revendication 1, présentant en outre au moins un élément (14) élastique, en particulier un ressort qui est comprimé à l'état fermé par une force de fermeture de la partie mâchoire (2) et lorsque la force de fermeture est supprimée, déplace la au moins une électrode métallique (8) et le au moins un corps de refroidissement (12) l'un par rapport à l'autre.

3. Instrument (1) selon la revendication 2, dans lequel le au moins un élément élastique (14) écarte la au moins une électrode métallique (8) et le au moins un corps de refroidissement (12) l'un de l'autre.

4. Instrument (1) selon l'une quelconque des revendications 1 à 3, dans lequel au moins une électrode métallique (8) et le au moins un corps de refroidissement (12) sont mobiles l'un par rapport à l'autre dans un sens de déplacement perpendiculaire à une étendue longitudinale des branches (4).

5. Instrument (1) selon l'une quelconque des revendications 1 à 4, dans lequel la au moins une électrode métallique (8) et le au moins un corps de refroidissement (12) présentent respectivement au moins une contre-dépouille, dans lequel les contre-dépouilles respectives sont espacées l'une de l'autre à l'état fermé et reposent l'une contre l'autre à l'état ouvert.

6. Instrument (1) selon la revendication 5, dans lequel la contre-dépouille limite le mouvement relatif entre la au moins une électrode métallique (8) et le au moins un corps de refroidissement (12) dans le sens de déplacement.

7. Instrument (1) selon l'une quelconque des revendications 1 à 6, dans lequel la au moins une électrode métallique (8) présente un corps d'électrode (16) avec la surface de contact (10) et une première denture (18) avec un nombre de premières dents (20) qui s'étendent depuis le corps d'électrode (16) dans le côté opposé à la surface de contact (10).

8. Instrument (1) selon la revendication 7, dans lequel les premières dents (20) de la première denture (18) présentent respectivement une première section de base (22) qui s'étend de préférence perpendiculairement au corps d'électrode (16) et une première section de tête (24), dans lequel la première section de tête (24) s'élargit depuis la première section de base (22) en forme de champignon parallèlement au corps d'électrode (16).

9. Instrument (1) selon l'une quelconque des revendications 1 à 8, dans lequel le au moins un corps de refroidissement (12) présente un corps de base (26) et une seconde denture (28) avec un nombre de secondes dents (30) qui s'étendent du corps de base (26) en direction de la au moins une électrode métallique (8).

10. Instrument (1) selon la revendication 9, dans lequel les secondes dents (30) présentent respectivement une seconde section de base (32) qui s'étend de préférence perpendiculairement au corps de base (26) et une seconde section de tête (34), dans lequel la seconde section de tête (34) s'élargit depuis la seconde section de base (32) en forme de champignon parallèlement au corps de base (26).

11. Instrument (1) selon la revendication 9 ou 10, dans lequel les premières dents (20) et les secondes dents (30) viennent en prise les unes dans les autres respectivement en alternance.

12. Instrument (1) selon la revendication 10 ou 11, dans lequel les premières dents (20) et les secondes dents (30) viennent en prise les unes dans les autres de sorte que les sections de tête (24, 34) en forme de champignon respectives reposent l'une contre l'autre à l'état ouvert et forment la contre-dépouille et soient espacées l'une de l'autre à l'état fermé.

13. Instrument (1) selon l'une quelconque des revendications 1 à 12, dans lequel au moins un élément d'écartement (36) qui est de préférence en forme de triangle est agencé au niveau de la au moins une électrode métallique (8) et/ou au niveau de l'au moins un corps de refroidissement (12).

14. Instrument (1) selon l'une quelconque des revendications 1 à 4, dans lequel la au moins une électrode métallique (8) est logée sur une bascule (38) de sorte que la au moins une électrode métallique (8) soit pivotée de l'état ouvert dans l'état fermé de l'au moins un corps de refroidissement (12) lors d'un déplacement des branches (4).

15. Instrument (1) selon la revendication 14, dans lequel une force de ressort de l'élément élastique (14) pivote la au moins une électrode métallique (8) dans l'état ouvert autour de la bascule (38) et la presse contre le au moins un corps de refroidissement (12).
